# EUROPEAN PATENT SPECIFICATION

(11) **EP 2 766 499 B1**
(45) Date of publication and mention of the grant of the patent: **09.08.2017**
(21) Application number: 12780691.7
(22) Date of filing: 12.10.2012
(51) Int. Cl.: C12Q 1/68, G01N 33/574

(54) **METHOD FOR DETECTION OF GALPHA15 AS TUMOR MARKER IN PANCREATIC CANCER**
VERFAHREN ZUM NACHWEIS VON G15 ALS TUMORMARKER BEI BAUCHSPEICHELDRÜSENKREBS
PROCÉDÉ POUR LA DÉTECTION DE G 15 EN TANT QUE MARQUEUR TUMORAL DANS LE CANCER DU PANCRÉAS

(30) Priority: 12.10.2011 IT PD20110324
(43) Date of publication of application: 20.08.2014
(73) Proprietor: Università Degli Studi Di Verona, 37129 Verona (IT); Innovation Factory S.r.l., 34149 Trieste (IT)
(72) Inventor: INNAMORATI, Giulio, I-37121 Verona (IT); BASSI, Claudio, I-37138 Chievo (IT); VALENTI, Maria Teresa, I-37030 Mezzane Di Sotto (IT); GIOVINAZZO, Francesco, I-37135 Verona (IT); DALLE CARBONARE, Luca, I-37030 Mezzane Di Sotto (IT)
(74) Representative: Zaccaro, Elisabetta
(86) International application number: PCT/EP2012/070290
(87) International publication number: WO 2013/053901

(56) References cited:
- WO-A1-2010/086388
- G Zanini: "The Heterotrimeric G15 Protein May Support Sustained PKD1 Activation", JOP. J Pancreas (Online) 2011 Oct 11; 12(5 Suppl), 10 October 2011 (2011-10-10), pages 502-556, XP055017043, Retrieved from the Internet: URL:http://www.joplink.net/prev/201109/138 .html [retrieved on 2012-01-20]
- Parenti: "Trasduzione del segnale operata dai GPCR mediante la proteina G15/16: un meccanismo per evadere la desensibilizzazione e generare segnali intracellulari continui?", DIMS 2007, 13 August 2008 (2008-08-13), XP055017050, Retrieved from the Internet: URL:http://www.dims.medicina.unimib.it/dow nload/Relazione%20DIMS%202007.pdf [retrieved on 2012-01-20]
- F. GIANNONE ET AL: "The puzzling uniqueness of the heterotrimeric G15 protein and its potential beyond hematopoiesis", JOURNAL OF MOLECULAR ENDOCRINOLOGY, vol. 44, no. 5, 11 February 2010 (2010-02-11), pages 259-269, XP055017143, ISSN: 0952-5041, DOI: 10.1677/JME-09-0134

## Description

### Field of the invention

The present invention relates to a method for the detection of Gα15 as a tumor marker in pancreatic cancer.

### State of the art

The pancreas is a gland consisting of an exocrine and an endocrine component, located deeply in the abdominal cavity. It is in close contiguity to the duodenum from which it originates and to which is connected by an excretory duct.

Pancreatic tumors can be classified as solid, cystic, and tumors with mixed components. These lesions may have different biological and histological characteristics corresponding to various degrees of aggressiveness, with very different prognosis. Among those, pancreatic cancer (PaCa) is the most aggressive form and sometimes occurs in association with tumors considered to be borderline. PaCa is the fourth leading cause of cancer death. Because of biological aggressiveness and diagnostic difficulties, PaCa remains an incurable disease.

To date, surgery represents the only therapeutic option. However, just 20% of patients are elegible and their average survival rate is less than 2 years, with few patients significantly exceeding this threshold.

In the majority of cases, the tumor is so advanced at the time of diagnosis that only palliative measures are available.

Therefore, despite the efforts made to define the molecular basis of the disease, the prognosis remains poor [1].

In order to develop, any tumor must subvert normal inter-cellular communications to evade the immune system, to obtain nutrients by stimulating angiogenesis, to colonize other organs during the metastatization process, etc.

3-5% of the human genome encodes G protein-coupled receptors (GPCRs) which are major players in inter-cellular communication under normal and pathological conditions, including cancer [2]. Heterotrimeric G proteins are the primary effectors, they mediate signal cascades regulating cell survival, transcription, cell motility etc. As for KRAS, mutations inactivating the GTPase domain can turn heterotrimeric G proteins in oncogenes [3].

Belonging to the Gq subfamily, the G15 protein activates phospholipase Cß [4], however, its physiological role remains substantially unknown [5]. Zanini S. et al 2011 disclose microarray data describing G15 expression in pancreas adenocarcinoma. Because of the location of the pancreas in the human body, early stages of pancreatic diseases remain very often asymptomatic until the tumor progresses to an advanced stage.

Current methods for diagnosis of this type of carcinoma have the disadvantages of involving invasive procedures unable to detect small size tumors or identify anatomical variants of some cancer forms.

The particular biological aggressiveness of PaCa and the difficulty to diagnose it at early stages underlie the lack of therapeutic approaches with curative intents.

Therefore, the purpose of the present invention is to provide a non-invasive method suitable for rapid and early diagnosis of pancreatic cancer and useful to provide prognostic indications during treatment, postoperative control and follow-up and which is devoid of the above mentioned disadvantages that characterize known methods for the same application.

### Summary of the invention

The invention relates to a method employing Gα15 as tumor marker for diagnosis of pancreatic cancer comprising the step of:
a) determining the presence of mRNA encoding for Gα15 in a biological sample, wherein the presence of said mRNA transcribed from the Gα15 gene indicates the presence of pancreatic carcinoma.

### DESCRIPTION OF THE FIGURES

The invention will now be described in detail and in reference to the attached Figures, wherein:
Figure 1 shows results quantifying the level of PKD1 activation by immunoblot with phosphospecific antibodies against S748 or S744. COS-7 cells were transfected with PKD1, Gα15 and V2 type vasopressin receptor (V2R-WT) or its constitutively desensitized mutant (V2R-R137H) as indicated in Example 1;
Figure 2 shows the results of ectopic expression of the mRNA encoding Gα15. mRNAs were extracted and quantified by reverse transcription reaction (cDNA) and subsequent amplification by Real Time PCR with highly specific and sensitive primers and probes (TaqMan probe).
Figure 2a) The presence of mRNA encoding for Gα15 was measured in cell lines derived from PaCa;
Figure 2b) The presence of mRNA encoding for Gα15 was measured in cellular subtypes derived from healthy pancreas;
Figure 2c) The presence of mRNA encoding for Gα15 was measured in xenotransplants obtained by implanting primary adenocarcinoma in athymic mice;

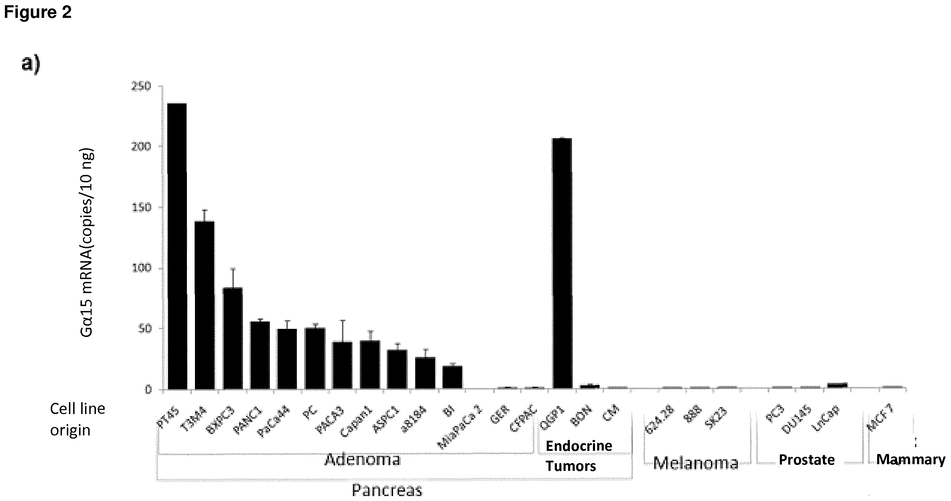

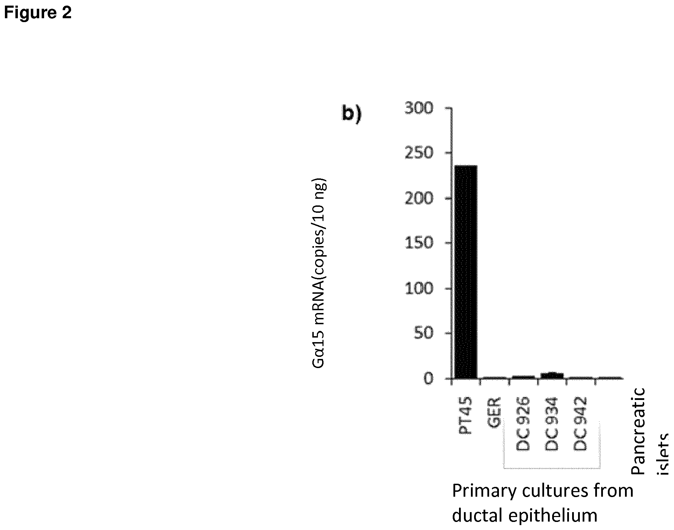

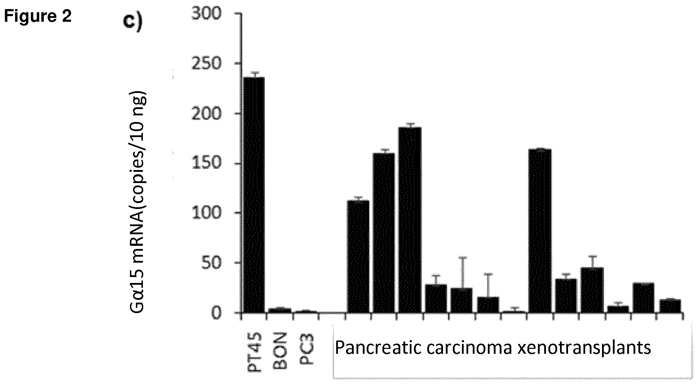

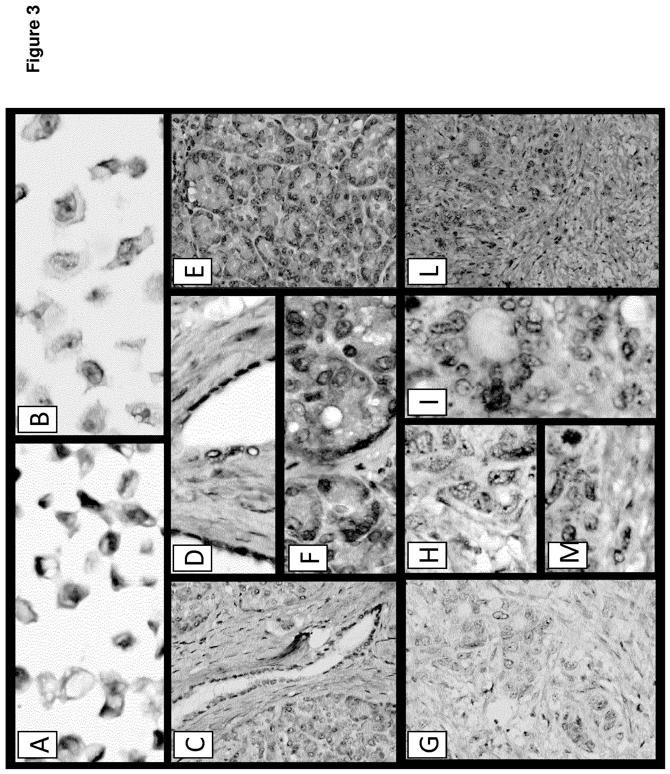

Figure 3 RNAscope detection of mRNA encoding for Gα15 in FFPE PaCa cells and tissues. Paraffin-embedded specimens sections were cut to a thickness of 4µm and mounted on positively charged glass slides. In-situ hybridization was performed utilizing a probe specific for Gα15 mRNA of SEQ ID NO:7. Representative images were captured from hematoxylin stained sections. Consistently with Real Time PCR data in Figure 2, Gα15 mRNA was absent in GER cells (A) and present (spots) in PT45 cells (B). Gα15 mRNA was substantially absent in duct (C,D) and acinar (E,F) cells and present in transformed areas (G-M). The stromal component of the tumor was substantially negative.
Figure 4 shows results related to the presence of Gα15 encoding mRNA in the serum from PaCa patients and healthy donors, as described in Example 5;
Figure 5 shows data and graphs related to increased Gα15-dependent resistance of PaCa cells in Example 3; lentiviral particles capable of producing shRNA or GFP were developed and tested on cell lines:
Figure 5a) the immunoblot shows efficient inhibition of Gα15 expression in PT45 and QGP1 cells for at least 4 of the 5 shRNA sequences utilized in the assay (64, 65, 66, 67 of SEQ ID NO:2, SEQ ID NO:3, SEQ ID NO:4 e SEQ ID NO:5, respectively);:
Figure 5b) two cell lines positive for Gα15 expression (QGP1 and PT45) and one cell line negative for Gα15 expression (GER) were grown in presence of 10% FCS. 3 days after treatment with viral particles encoding shRNA, as in fig.4a; cells were stained with crystal violet.
Figure 5c) as in b) except FCS was removed from the growth medium at day 2. Cell number in b) and c) was digitally quantified using NIH Image J; values are expressed as % of GFP transduced cells, ± S.E.M. n=7 *p<0.05 **p <0.01.
Figure 5d) Cells were treated with shRNA-encoding viral particles as in fig.4a and incubated for 21 days in suspension ("under-agar assay"). The number of colonies formed from individual PT45 and GER cells was quantified under the microscope. Values are expressed as % of GFP transduced cells ±S.E.M. n=3 **p <0.01.

### Detailed description of the invention

Therefore, the invention relates to a method for detecting Gα15 as a tumor marker for pancreatic carcinoma comprising the step of:
a) determining the presence of mRNA encoding for Gα15 in a biological sample, wherein the presence of said mRNA transcribed from the Gα15 gene (mRNA encoding for Gα15) indicates the presence of pancreatic carcinoma.

For the purpose of the present invention the definition:
- "mRNA" is meant to comprise a type of RNA, or "messenger RNA", also known by the more generic term of transcript, which encodes and carries information from DNA to the sites of protein synthesis, in order to be translated; and
- "Gα15" ("guanine nucleotide binding protein (G protein), alpha 15 (Gq class") is meant to indicate a heterotrimeric G protein (encoded by the GNA15 gene) belonging to the subfamily of Gq proteins, with Gene ID: 2769 and for example NCBI Reference Sequences (RefSeq) NM_002068.2 and NP_002059.2.

In fact, as it will be evident from the Examples below, such method has proved surprisingly effective for direct assessment of the presence of mRNA encoding for Gα15 in biological samples and allows early diagnosis of the disease.

Moreover, this method allows, through the detection of mRNA encoding for Gα15, to offer prognostic indications during treatment, postoperative control, and in the course of follow-up.

Another aspect of the invention relates to a method for detecting the presence of mRNA encoding for Gα15 in a biological sample selected from the group consisting of a sample of blood, saliva, serum, postoperative drainage, pancreatic juice, urine, cystic fluid and faeces (stool) Conveniently, in a preferred realization said biological sample is a serum sample. Blood serum results from a fluid formed by plasma (the liquid phase of blood) without fibrinogen.

The method according to the present invention has the advantage of allowing non-invasive detection of mRNA encoding for Gα15 in biological samples such as blood serum, which represents the best choice for patients. In addition, the greater sensitivity of the method allows detection, in a single analysis, of the presence or absence of mRNA encoding for Gα15, with consequent cost reduction and time saving.

In a further preferred realization said biological sample is a biopsy sample from resection, laparoscopy or needle aspiration. Biopsy is a medical examination consisting in removal from a living organism of a portion or fragment of tissue and subsequent analysis.

According to a preferred realization, in said method for diagnosis of pancreatic carcinoma, the step for determining the presence of mRNA encoding for Gα15 is carried out by techniques suitable for measuring the presence of nucleic acids.

In particular, such techniques can measure the products of the specific mRNA sequence, however the method is meant to be extendable to the possibility of quantifying products of said sequence, such as for example, the protein product or even degradation products of the mRNA itself.

According to a further preferred realization, in said method for the diagnosis of pancreatic carcinoma, the step of determining the presence of mRNA encoding for Gα15 mRNA is carried out by RT Real-Time PCR. The real-time PCR, also known as quantitative PCR or quantitative PCR in real time, is a method for amplification and simultaneous quantification of DNA or, as in the present case, of cDNA (complementary DNA) as it is obtained by reverse transcription (RT) of mRNA. The Real-Time PCR technique has many advantages, for example it allows to obtain results in a short time, can be conveniently carried out with small volumes hence keeping the costs low.

Conveniently, in one realization said Real-Time PCR is performed using a probe capable of detecting specifically GNA15.

Said probe conveniently comprises the sequence:
SEQ ID No: 1 GCTGCTGCTTTTGGGCCCAGGCGAG.

According to a still further preferred realization, in said method for the diagnosis of pancreatic carcinoma, the step of determining the presence of mRNA encoding for Gα15 is carried out by *in situ* hybridization.

In a further example the specification relates to Gα15 inhibitors. Such inhibitors are shRNA sequences which block Gα15 function by gene silencing with lentiviral particles encoding for a Gα15-specific shRNA sequence or by techniques based on siRNA or any other molecule which pharmacologically targets Gα15 or other proteins involved in its function. Since Gα15 supports cancer cells in hostile conditions typical of the tumoral microenvironment (i.e. lack of nutrients and anchorage), such inhibitors could be exploited to downmodulate Gα15 and thus specifically weaken cancer cells.

Advantageously Gα15 inhibitors can be utilized for the treatment of pancreatic cancer, alone or in association with other treatments based on different strategies: pharmacological treatment (see gemcitabine or oxaliplatin), radiofrequency ablation or surgical resection.

According to a preferred realization, the differential diagnosis of pancreatic cancer according to the method of the present invention is made among pathologies selected from the group consisting of pancreatic adenocarcinoma, serous cystadenoma, mucinous cytoadenoma, mucinous cystadenocarcinoma, intraductal papillary mucinous neoplasm, acinar cell cancer, neuroendocrine pancreatic tumors, pseudopapillary neoplasia, cystic teratoma, cystic choriocarcinoma, angiomatous tumors, pancreatic pseudocyst, wherein more preferably said carcinoma is pancreatic adenocarcinoma

Surgery is the only treatment for localized adenocarcinomas, optionally associated with adjuvant chemo/radiotherapy, whereas locally advanced adenocarcinomas are candidates for chemo/radiotherapy and may subsequently be treated surgically if operability is achieved; in the case of advanced tumors the only treatment is palliative chemo/radiotherapy.

Approximately 95% of pancreatic cancers are adenocarcinomas. The remaining 5% include other exocrine pancreas tumors (e.g. serous cystadenoma), acinar cell cancer, and pancreatic neuroendocrine tumors (such as insulinomas).

In a further aspect, the method according to the present invention further comprises step b) wherein the biological sample is monitored over time according to step a) in order to assess the effect of therapeutic treatments received by the patient.

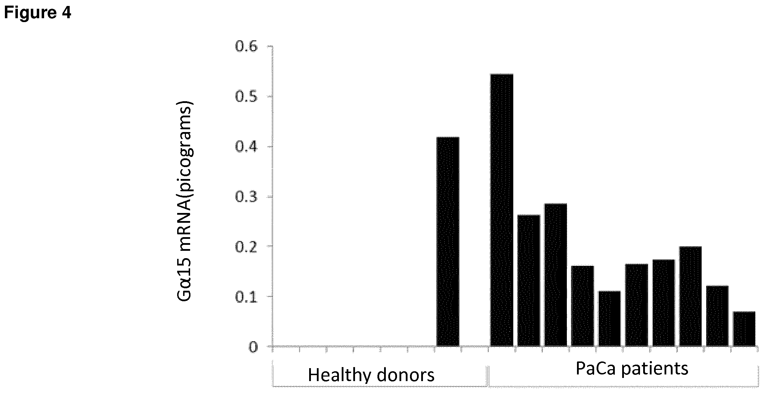

A particular application of the method according to the present invention consists in assessing the disappearance from the organism of Gα15 expressing cells. According to a preferred realization, monitoring as intended in step b) is applied during follow-up of patients treated with therapies based on silencing with lentiviral particles encoding for a Gα15-specific shRNA sequence (see example in Fig. 4), or on other techniques based on siRNA or any other molecule which pharmacologically targets Gα15 or other proteins involved in its function. For effectiveness of treatment it is meant the therapeutic effect linked to a reduction of tumor cell number in the organism.

In the present invention the definition :
- "silencing the gene" is meant to include a mechanism allowing inhibition of the expression of individual genes,
- "shRNA" or short hairpin RNA is meant to include an RNA sequence that is usually employed for silencing gene expression;
- "siRNA" or small/short interfering RNA is a 20 to 25 nucleotides long RNA molecule capable of performing numerous biological roles and is primarily involved in the RNA interference pathway leading to inhibition of the expression of individual genes ; and
- "Lentiviral particles encoding a shRNA sequence" refers to viruses that allow transfer of genetic information, in the form of vectors and plasmids, to the host cell DNA. The lentiviral particles encoding a shRNA sequence allow the transfer of one or more plasmids or shRNA encoding vectors in a target cell.

In a preferred realization, the shRNA sequence specific for Gα15 is conveniently selected from the group consisting of:
- SEQ ID No:2, NM_002068.1-580s1c1
- SEQ ID No:3, NM_002068.1-981s1c1
- SEQ ID No:4 NM 002068.1-879s1c1
- SEQ ID No:5. NM_002068.1-684s1c1
- SEQ ID No:6. NM_002068.1-1143s1c1

Examples of realization of the present invention are provided below for illustrative purposes.

### EXAMPLES

### Example 1: Functional features of the Gα15 protein

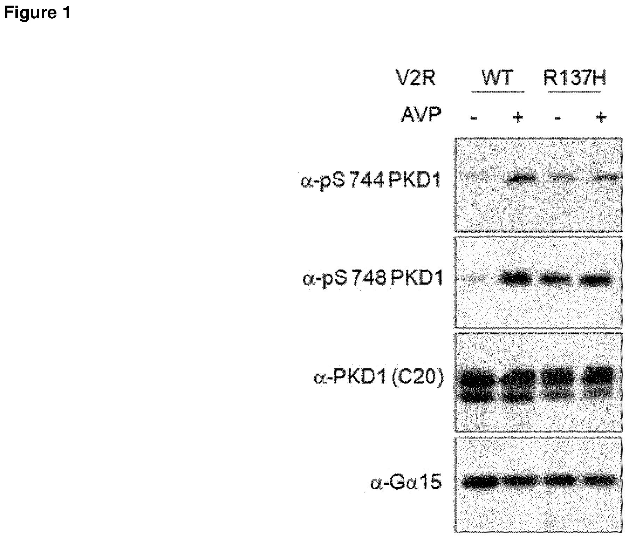

The R137H mutation locks the vasopressin V2 receptor (V2R-WT) in an active conformation simulating the interaction with the agonist. Thus, newly synthesized V2R-R137H is heavily phosphorylated and it is complexed with β-arrestin, forced in a "constitutively desensitized" state even before reaching the plasma membrane [8]. As such, the R137H mutation causes nephrogenic diabetes insipidus. In response to vasopressin (AVP), V2R-WT activates co-transfected Gαq and Gα15. V2R-R137H is instead incapable of activating co-transfected Gαq, but if Gα15, is co-transfected, it is functionally active both in presence and absence of AVP [7]. Thus Gα15 co-expression recovers the functionality of desensitized V2R-R137H. To analyze the impact on downstream effectors, COS-7 cells were transfected with PKD1, Gα15 and V2R-WT or the constitutively desensitized mutant (V2R-R137H). PKD1 activation was quantified by immunoblots with phosphospecific antibodies directed against S748 or S744. Figure 1 shows that PKD1 activity depends on AVP in the case V2R-WT, whereas V2R-R137H (normally silent even in presence of AVP) acts as a constitutively active receptor and promotes the activity of an effector downstream of phospholipase Cβ, like PKD1, even in the absence of agonist. Thus, Gα15 collects the signals produced by desensitized GPCR transferring them to the nucleus and to other sites.

### Example 2: Screening for potential PaCa markers and in situ hybridization

Gα15 expression profile is extremely restricted (hematopoietic cells, keratinocytes and epithelial cells of the thymus) [5].

Since Gα15 is absent in most tissues of our body, in order to play to play an active role in neoplastic transformation it must become ectopically expressed by the tumor.

A screening based on 'global gene expression technology' identified Gα15 among 97 potential PaCa markers [9]. In a screening on cultured cell lines, we determined that Gα15 (fig. 2a) is particularly expressed in cell lines derived from pancreatic carcinomas.

In a screening of resected samples we determined Gα15 expression in the neoplastic fraction of tumor biopsies from PaCa and its absence in normal pancreatic samples, including primary ductal cell cultures or pancreatic islets (fig.2b). Adenocarcinoma samples transplanted in nude mice were found to be markedly positive by using a TaqMan probe specific for the human Gα15 ortholog (Fig. 2c). This implies that the positive signal is generated from cells that propagate the tumor rather than surrounding cells of the host (stroma, infiltrate, etc..).

### In situ hybridization

In situ hybridization was performed utilizing RNAscope (Advanced Cells Diagnostics) on paraffin-embedded specimens. The assay was initially validated on cell lines. As a negative control (Figure 3, panel A), we utilized GER cells, the cell expressing lower Gα15 mRNA levels in figure 2a. As a positive control (Figure 3, panel B), we utilized PT45 cells, the cell expressing higher Gα15 mRNA levels in figure 2a.

The assay was next utilized to analyze transformed pancreas. Tissue adjacent to the tumor, but showing no signs of transformation, appeared as negative in the ducts (Figure 3, panels C and D) and in the acinar region (Figure 3, panels E and F). This result is consistent with the data obtained by rt-PCR in figure 2b and with data present in the literature claiming that Gα15 is not expressed in pancreas. As opposite, the transformed region (Figure 3, panels G to L) appears positive. In particular, the signal is distributed in the neoplastic epithelium, while the surrounding desmoplastic stroma does not label.

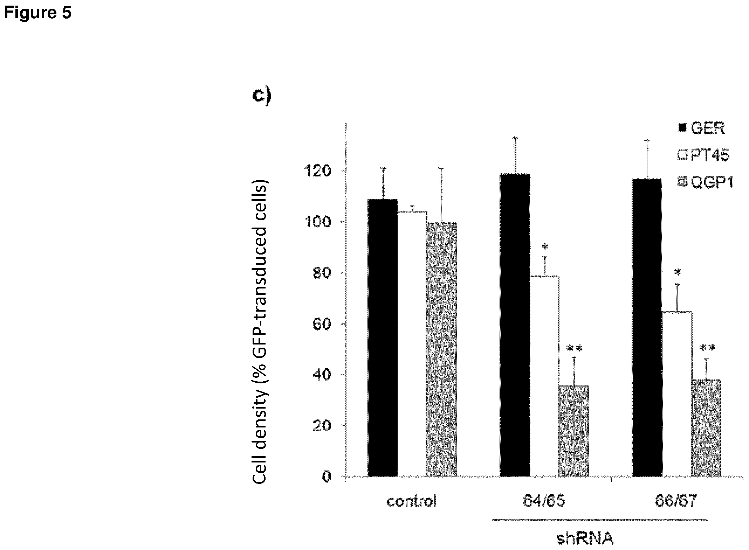

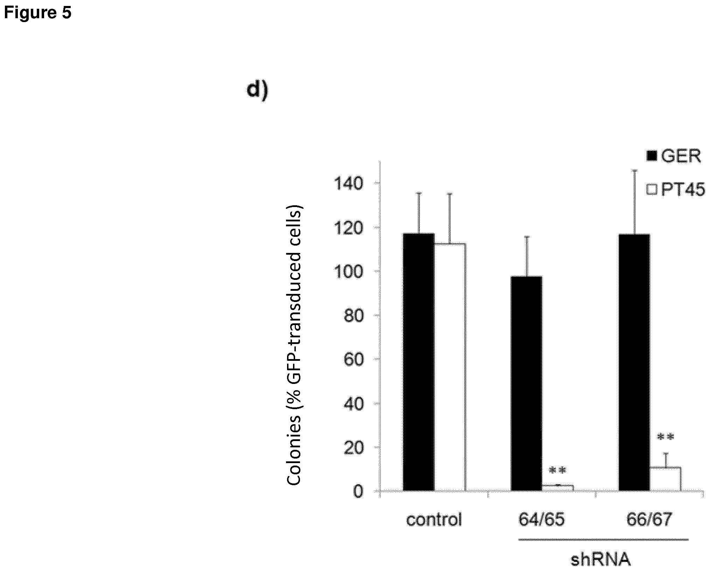

Based on the results shown in particular in Figure 5c and d, Gα15 supports cancer cells in hostile conditions typical of the tumoral microenvironment (i.e. lack of nutrients and anchorage).

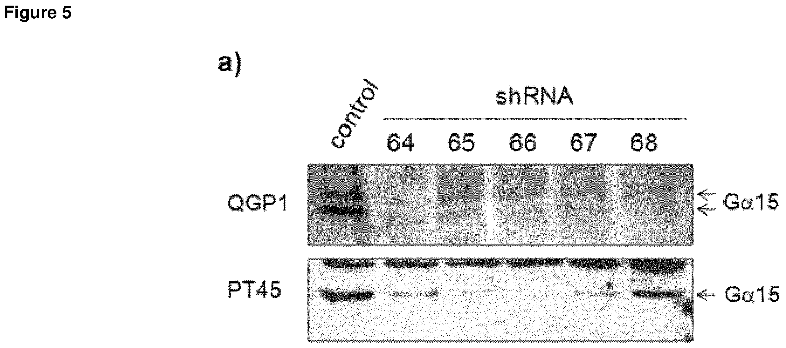

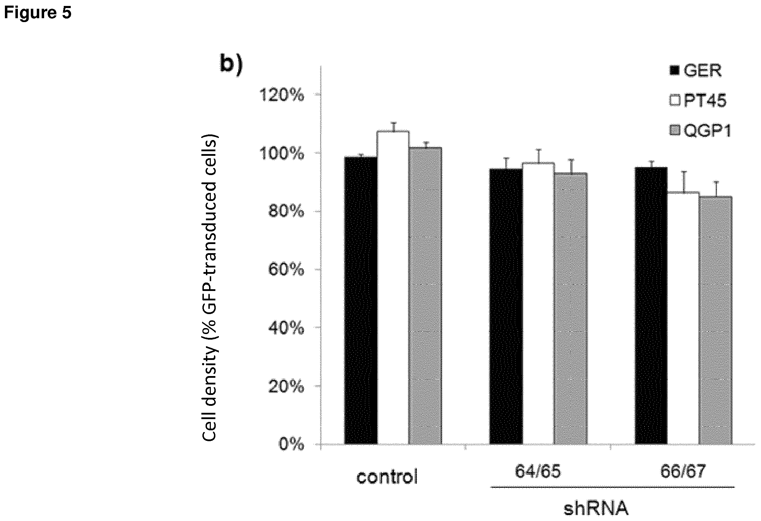

Sequences like those utilized in the assay described in fig. 5 could be exploited to downmodulate Gα15 and thus specifically weaken cancer cells.

### Example 3: Analysis of Gα15 presence in cell lines derived from human PaCa

Using RT-PCR and immunoblot, we have documented the presence of Gα15 in human PaCa cell lines including PT45 (derived from adenocarcinoma) and QGP1 (derived from carcinoma of pancreatic islets) (fig. 2a, 4a). In these cellular models, the function of Gα15 was specifically inhibited by use of lentiviral particles encoding 5 shRNA sequences, all of them specific for Gα15. In 4 of 5 cases, Gα15 expression was reduced by more than 80% in both PT45 and QGP1 cells (see fig. 4a). No effect was instead produced by a control shRNA sequence (similar but random sequence) or a "green fluorescent protein" (GFP) encoding sequence.

In PaCa cells (PT45, QGP1), silencing Gα15 reduced resistance to lack of nutrients and anchorage independent growth ("under agar assay"). In fact, a significant reduction in cell number was observed with both shRNA combinations used to silence Gα15 expression (fig.5c and 5d respectively), while no effect was observed in PaCa cells lacking Gα15 expression (GER).

### Example 4: Process for detecting the presence of mRNA encoding for Gα15 in serum

- 1 ml of blood is collected maintaining sterility and avoiding contamination from skin.
- Serum extraction.
- mRNA purification (Quiamp ultrasense virus kit, Qiagen).
- Reverse transcription (from mRNA to cDNA) (Superscript III RT, Invitrogen).
- Quantification by TaqMan PCR using a GNA15 specific probe
   GCTGCTGCTTTTGGGCCCAGGCGAG (SEQ ID NO:1) (Taqman, Appliled Biosystem)

Experiments made using the process described above show the increased presence of Gα15 encoding mRNA in the serum of PaCa patients (fig.4). The results show very high specificity (87.5%) and sensitivity (100%) values compared to other tumor markers. The technique carried out with limited amount of serum is associated with low invasiveness.

### Example 5: Analysis of mRNA encoding for Gα15 in other biopsy specimens of PaCa patients.

The same process of Example 3 can be applied to other biopsy specimens (postoperative drainage, saliva, urine, cystic fluid, pancreatic juice, faeces).

### Example 6: Process for detecting the presence of mRNA encoding for Gα15 in the tumor

- mRNA extraction from intraoperatively resected specimens.
- Reverse transcription (from mRNA to cDNA) (Superscript Invitrogen).
- Quantification by TaqMan PCR using a GNA15 specific probe GCTGCTGCTTTTGGGCCCAGGCGAG (SEQ ID NO:1).

The same process could be applied to other biopsy specimens containing tumor cells (resection, laparoscopy, needle aspiration).

The advantages achieved by the method of the present invention are evident from the detailed description and from the above Examples. In particular, this method proved to be surprisingly and conveniently suitable for non-invasive diagnosis of pancreatic carcinoma. At the same time, as this method is fast and extremely easy to perform, it can be conveniently carried out in any type of laboratory.

From the above description and the above-noted Examples, the advantages attained and obtained according to the present invention are apparent.

### BIBLIOGRAPHY

1. Ghaneh, P.; Costello, E.; Neoptolemos, J. P., Biology and management of pancreatic cancer. Postgrad Med J 2008, 84, 478-497.
2. Dorsam, R. T.; Gutkind, J. S., G-protein-coupled receptors and cancer. Nat.Rev.Cancer 2007, 7, 79-94.
3. Van Raamsdonk, C. D.; Bezrookove, V.; Green, G.; Bauer, J.; Gaugler, L.; O'Brien, J. M.; Simpson, E. M.; Barsh, G. S.; Bastian, B. C., Frequent somatic mutations of GNAQ in uveal melanoma and blue naevi. Nature 2009, 457, 599-602.
4. Hubbard, K. B.; Hepler, J. R., Cell signalling diversity of the Gqalpha family of heterotrimeric G proteins. Cell Signal 2006, 18, 135-150.
5. Giannone, F.; Malpeli, G.; Lisi, V.; Grasso, S.; Shukla, P.; Ramarli, D.; Sartoris, S.; Monsurro, V.; Krampera, M.; Amato, E.; Tridente, G.; Colombatti, M.; Parenti, M.; Innamorati, G., The puzzling uniqueness of the heterotrimeric G15 protein and its potential beyond hematopoiesis. J Mol Endocrinol 2010, 44, 259-269.
6. Milligan, G.; Marshall, F.; Rees, S., G16 as a universal G protein adapter: implications for agonist screening strategies. Trends Pharmacol.Sci. 1996, 17, 235-237.
7. Innamorati, G.; Giannone, F.; Guzzi, F.; Rovati, G. E.; Accomazzo, M. R.; Chini, B.; Bianchi, E.; Schiaffino, M. V.; Tridente, G.; Parenti, M., Heterotrimeric G proteins demonstrate differential sensitivity to beta-arrestin dependent desensitization. Cell Signal. 2009, 21, 1135-1142.
8. Wilbanks, A. M.; Laporte, S. A.; Bohn, L. M.; Barak, L. S.; Caron, M. G., Apparent loss-of-function mutant GPCRs revealed as constitutively desensitized receptors. Biochemistry 2002.Oct.8.;41 .(40.):11981 .-9. 2002, 41, 11981-11989.
9. lacobuzio-Donahue, C. A.; Maitra, A.; Shen-Ong, G. L.; van Heek, T.; Ashfaq, R.; Meyer, R.; Walter, K.; Berg, K.; Hollingsworth, M. A.; Cameron, J. L.; Yeo, C. J.; Kern, S. E.; Goggins, M.; Hruban, R. H., Discovery of novel tumor markers of pancreatic cancer using global gene expression technology. Am J Pathol 2002, 160, 1239-1249.

## Claims

1. Method for detecting Gα15 as a marker for pancreatic cancer comprising the steps of:
a. determining the presence of mRNA encoding for Gα15 in a biological sample, wherein the presence of said mRNA encoding Gα15 is an indication of pancreatic cancer, wherein said biological sample is chosen from the group consisting of a blood sample, serum, saliva, urine, cystic fluid, postoperative drainage, pancreatic juice and faeces.

2. Method according to claim 1, wherein said biological sample is a sample of serum.

3. Method according to anyone of claims 1 to 2, wherein said step of determining the presence of mRNA encoding Gα15 is carried out by a technique which measures the presence of nucleic acids.

4. Method according to anyone of claims 1 to 2, wherein said step of determining the presence of mRNA encoding Gα15 is carried out by a technique suitable for measuring the products of a specific mRNA sequence.

5. Method according to claim 4, wherein said technique which measures the products of a specific mRNA sequence is RT Real-Time PCR.

6. Method according to claim 5, wherein said RT Real-Time PCR technique is carried out with a probe which specifically detects mRNA encoding Gα15.

7. Method according to anyone of claims 1 to 6, wherein said pancreatic cancer is chosen from the group consisting of pancreatic adenocarcinoma, serous cystadenoma, mucinous cytoadenoma, mucinous cystadenocarcinoma, intraductal papillary mucinous neoplasm, acinar cell cancer, pancreatic neuroendocrine tumor, pseudopapillary tumor, cystic teratoma, choriocarcinoma, cystic angiomatous tumors, pancreatic pseudocysts.

8. Method according to claim 7, wherein said pancreatic cancer is a pancreatic adenocarcinoma.

9. Method according to anyone of claims 1 to 8, further comprising a step b. in which the biological sample encoding Gα15 mRNA of step a. is collected again from the patient for monitoring the presence or the absence of tumor cells in the organism.

10. Method according to claim 9, wherein the absence of tumor cells from the organism is due to a therapy having Gα15 as a pharmacological target.

## Patentansprüche

1. Verfahren zum Nachweis von Gα 15 als Tumormarker bei Bauchspeicheldrüsenkrebs, aufweisend die folgende Schritte:
a.) Ermittlung der Anwesenheit einer mRNA-Kodierung von Gα15 in einer biologischen Probe, wobei die Anwesenheit einer mRNA-Kodierung von Gα15 ein Hinweis auf Bauchspeicheldrüsenkrebs ist und wobei die biologische Probe ausgewählt wird aus der Gruppe bestehend aus einer Blutprobe, Serum, Speichel, Urin, Zystenflüssigkeit, postoperativer Drainage, Pankreassaft und Kot.

2. Verfahren gemäß Anspruch 1, wobei die biologische Probe eine Serumsprobe ist.

3. Verfahren gemäß einem der Ansprüche 1 bis 2, wobei der Schritt der Ermittlung der Anwesenheit einer mRNA-Kodierung von Gα15 mittels einer Technik durchgeführt wird, die die Anwesenheit von Nukleinsäure misst.

4. Verfahren gemäß einem der Ansprüche 1 bis 2, wobei der Schritt der Ermittlung der Anwesenheit einer mRNA-Kodierung von Gα15 mittels einer Technik durchgeführt wird, die geeignet ist Produkte einer bestimmten mRNA-Sequenz zu messen.

5. Verfahren gemäß Anspruch 4, wobei die Technik, die Produkte einer bestimmten mRNA-Sequenz zu messen, Echtzeit-PCR ist.

6. Verfahren gemäß Anspruch 5, wobei die Echtzeit-PCR-Technik mit einer Probe durchgeführt wird, die speziell mRNA-Kodierung von Gα15 nachweist.

7. Verfahren gemäß einem der Ansprüche 1 bis 6, wobei der Bauchspeicheldrüsenkrebs aus der Gruppe bestehend aus Pankreaskarzinom, serösem Zystadenom, muzinösem Zytoadenom, muzinösem Zystadenokarzinom, intraduktalem papillärem muzinösem Tumor, Azinuszellenkrebs, neuroendokrinem Tumor der Bauchspeicheldrüse, pseudopapillärem Tumor, zystischem Teratom, Chorionkarzinom, zystischem angiomatösem Tumor, pankreatischer Pseudozyste ausgewählt wird.

8. Verfahren gemäß Anspruch 7, wobei der Bauchspeicheldrüsenkrebs ein Pankreaskarzinom ist.

9. Verfahren gemäß einem der Ansprüche 1 bis 8, das zudem einen Schritt b.) aufweist, in dem die biologische Probe mit der mRNA-Kodierung von Gα15 aus Schritt a.) nochmals vom Patienten genommen wird, um die Anwesenheit oder Abwesenheit von Tumorzellen im Organismus zu überprüfen.

10. Verfahren gemäß Anspruch 9, wobei die Abwesenheit von Tumorzellen im Organismus aufgrund einer Therapie, welche Gα15 als pharmakologischem Ziel aufweist.

## Revendications

1. Procédé de détection de Gα15 en tant que marqueur de cancer du pancréas, comprenant les étapes consistant à :
a. déterminer la présence d'ARNm codant Gα15 dans un échantillon biologique, où la présence dudit ARNm codant Gα15 est une indication de cancer du pancréas, ledit échantillon biologique étant choisi dans le groupe constitué par un échantillon de sang, du sérum, de la salive, de l'urine, un fluide kystique, un liquide de drainage postopératoire, du suc pancréatique et des fèces.

2. Procédé selon la revendication 1, dans lequel ledit échantillon biologique est un échantillon de sérum.

3. Procédé selon l'une quelconque des revendications 1 à 2, dans lequel ladite étape de détermination de la présence d'ARNm codant Gα15 est réalisée au moyen d'une technique qui mesure la présence d'acides nucléiques.

4. Procédé selon l'une quelconque des revendications 1 à 2, dans lequel ladite étape de détermination de la présence d'ARNm codant Gα15 est réalisée au moyen d'une technique adaptée à la mesure des produits d'une séquence d'ARNm spécifique.

5. Procédé selon la revendication 4, dans lequel ladite technique qui mesure les produits d'une séquence d'ARNm spécifique est une RT-PCR en temps réel.

6. Procédé selon la revendication 5, dans lequel ladite technique de RT-PCR en temps réel est réalisée avec une sonde qui détecte spécifiquement un ARNm codant Gα15.

7. Procédé selon l'une quelconque des revendications 1 à 6, dans lequel ledit cancer du pancréas est choisi dans le groupe constitué par un adénocarcinome du pancréas, un cystadénome séreux, un cystadénome mucineux, un cystadénocarcinome mucineux, une tumeur intracanalaire papillaire et mucineuse, un carcinome à cellules acineuses, une tumeur neuroendocrine du pancréas, une tumeur pseudopapillaire, un tératome kystique, un choriocarcinome, les tumeurs angiomateuses kystiques, les pseudokystes pancréatiques.

8. Procédé selon la revendication 7, dans lequel ledit cancer du pancréas est un adénocarcinome du pancréas.

9. Procédé selon l'une quelconque des revendications 1 à 8, comprenant en outre une étape b. dans laquelle l'échantillon biologique contenant de l'ARNm codant Gα15 de l'étape a. est recueilli une nouvelle fois chez le patient pour contrôler la présence ou l'absence de cellules tumorales dans l'organisme.

10. Procédé selon la revendication 9, dans lequel l'absence de cellules tumorales dans l'organisme est due à une thérapie ayant Gα15 comme cible pharmacologique.


### SEQUENCE LISTING

<110> INNOVATION FACTORY S.R.L.
   UNIVERSITA' DEGLI STUDI DI VERONA
<120> METHOD FOR DETECTION OF Galfa15 AS TUMOR MARKER IN PANCREATIC CANCER
<130> 11293PTWO
<150> ITPD2011A000324
   <151> 2011-10-12
<160> 7
<170> BiSSAP 1.0
<210> 1
   <211> 25
   <212> DNA
   <213> Homo sapiens
<220>
   <221> source
   <222> 1..25
   <223> /mo1_type="DNA" /organism="Homo sapiens"
<400> 1
   gctgctgctt ttgggcccag gcgag 25
<210> 2
   <211> 58
   <212> DNA
   <213> Homo sapiens
<220>
   <221> source
   <222> 1..58
   <223> /mol_type="DNA" /organism="Homo sapiens"
<400> 2
   ccggccctat aaagtgacca cgtttctcga gaaacgtggt cactttatag ggtttttg 58
<210> 3
   <211> 58
   <212> DNA
   <213> Homo sapiens
<220>
   <221> source
   <222> 1..58
   <223> /mol_type="DNA" /organism="Homo sapiens"
<400> 3
   ccggcctcgc attgtttggg actatctcga gatagtccca aacaatgcga ggtttttg 58
<210> 4
   <211> 58
   <212> DNA
   <213> Homo sapiens
<220>
   <221> source
   <222> 1..58
   <223> /mol_type="DNA" /organism="Homo sapiens"
<400> 4
   ccggccattg tttcgagaac gtgatctcga gatcacgttc tcgaaacaat ggtttttg 58
<210> 5
   <211> 58
   <212> DNA
   <213> Homo sapiens
<220>
   <221> source
   <222> 1..58
   <223> /mol_type="DNA" /organism="Homo sapiens"
<400> 5
   ccggcctgct cgattcagcc gtgtactcga gtacacggct gaatcgagca ggtttttg 58
<210> 6
   <211> 58
   <212> DNA
   <213> Homo sapiens
<220>
   <221> source
   <222> 1..58
   <223> /mol_type="DNA" /organism="Homo sapiens"
<400> 6
   ccggcaagag gttcatcctg gacatctcga gatgtccagg atgaacctct tgtttttg 58
<210> 7
   <211> 1498
   <212> DNA
   <213> Homo sapiens
<220>
   <221> source
   <222> 1..1498
   <223> /mol_type="DNA" /note="In-situ hybridization probe specific for Galfa15 mRNA" /organism="Homo sapiens"
<400> 7
   cctggtctac cagaacatct tcgtgtccat gcgggccatg atcgaggcca tggagcggct 60
   gcagattcca ttcagcaggc ccgagagcaa gcaccacgct agcctggtca tgagccagga 120
   cccctataaa gtgaccacgt ttgagaagcg ctacgctgcg gccatgcagt ggctgtggag 180
   ggatgccggc atccgggcct actatgagcg tcggcgggaa ttccacctgc tcgattcagc 240
   cgtgtactac ctgtcccacc tggagcgcat caccgaggag ggctacgtcc ccacagctca     300
   ggacgtgctc cgcagccgca tgcccaccac tggcatcaac gagtactgct tctccgtgca     360
   gaaaaccaac ctgcggatcg tggacgtcgg gggccagaag tcagagcgta agaaatggat     420
   ccattgtttc gagaacgtga tcgccctcat ctacctggcc tcactgagtg aatacgacca     480
   gtgcctggag gagaacaacc aggagaaccg catgaaggag agcctcgcat tgtttgggac     540
   tatcctggaa ctaccctggt tcaaaagcac atccgtcatc ctctttctca acaaaaccga     600
   catcctggag gagaaaatcc ccacctccca cctggctacc tatttcccca gtttccaggg     660
   ccctaagcag gatgctgagg cagccaagag gttcatcctg gacatgtaca cgaggatgta     720
   caccgggtgc gtggacggcc ccgagggcag caagaagggc gcacgatccc gacgcctctt     780
   cagccactac acatgtgcca cagacacaca gaacatccgc aaggtcttca aggacgtgcg     840
   ggactcggtg ctcgcccgct acctggacga gatcaacctg ctgtgaccca ggccccacct     900
   ggggcaggcg gcaccggcgg gcgggtggga ggtgggagtg gctgcaggga cccctagtgt     960
   ccctggtcta tctctccagc ctcggcccac acgcaaggga gtcgggggac ggacggcccg    1020
   ctgctggccg ctctcttctc tgcctctcac caggacagcc gccccccagg gtactcctgc    1080
   ccttgcttga ctcagtttcc ctcctttgaa agggaaggag caaaacggcc atttgggatg    1140
   ccagggtgga tgaaaaggtg aagaaatcag gggattgagg acttgggtgg gtgggcatct    1200
   ctcaggagcc ccatctccgg gcgtgtcacc tcctgggcag ggttctggga ccctctgtgg    1260
   gtgacgcaca ccctgggatg gggctagtag agccttcagg cgccttcggg cgtggactct    1320
   ggcgcactct agtggacagg agaaggaacg ccttccagga acctgtggac taggggtgca    1380
   gggacttccc tttgcaaggg gtaacagacc gctggaaaac actgtcactt tcagagctcg    1440
   gtggctcaca gcgtgtcctg ccccggtttg cggacgagag aaatcgcggc ccacaagc      1498